Europäisches Patentamt

European Patent Office

Office européen des brevets

(11) Publication number: **0 154 186 B1**

## EUROPEAN PATENT SPECIFICATION

(45) Date of publication of patent specification: **22.07.92**    (51) Int. Cl.⁵: **C12Q 1/68**, C12N 15/10, C12N 15/21

(21) Application number: **85101283.1**

(22) Date of filing: **07.02.85**

(54) **Isolation of a novel interferon gene and expression thereof.**

(30) Priority: **10.02.84 US 579024**
**03.07.84 US 626591**

(43) Date of publication of application:
**11.09.85 Bulletin 85/37**

(45) Publication of the grant of the patent:
**22.07.92 Bulletin 92/30**

(84) Designated Contracting States:
**CH DE FR GB LI**

(56) References cited:
**EP-A- 0 089 692      EP-A- 0 128 042**
**EP-A- 0 159 714      WO-A-84/01153**
**WO-A-85/02862      FR-A- 2 541 579**

**AM J. Hum. Genet., vol. 31, 1979, pages 531-538, Am. Soc. of Hum. Genet. US AD Riggs et al "Synthetic DNA and medicine**

(73) Proprietor: **Wadley Technologies, Inc.**
**9000 Harry Hines Boulevard**
**Dallas, Texas 75235(US)**

(72) Inventor: **Bollon, Arthur Peter**
**3919 Rosser Square**
**Dallas Texas(US)**
Inventor: **Fuke, Motohiro**
**4522 East 103rd Street South**
**Tulsa Oklahoma(US)**
Inventor: **Torczynski, Richard Michael**
**24247 Shoredale**
**Farmers Branch Texas(US)**

(74) Representative: **UEXKÜLL & STOLBERG Patentanwälte**
**Beselerstrasse 4**
**W-2000 Hamburg 52(DE)**

Rank Xerox (UK) Business Services

Proceedings of the National Academy of Sciences of the USA, vol. 80., n 22, November 1983, pages 6838-6842, Washington, US. S. Anderson et al "Isolation of a genomic clone for bovine pancreatic trypsin inhibitor by using a unique-sequence synthetic DNA probe".

Chemical abstracts, vol. 99, n 9, August 1983, page 168, ref. n 65253y, Columbus, Ohio, US. E. Grens et al "New type of leukocytic interferon" & Dokl. Akad. Nauk SSSR 1983, 269(4), 986-90

Patent abstract of Japan, vol. 9, n 196, August 13, 1985, page 149 C 297 & JP A 6066992 (Wakunaga Seiyaku K.K.) 17.4.85

Chemical abstracts, vol. 102, n 19, May1985, page 155, n 161416e, Columbus, Ohio, US. A.P. Bollon et al "Homology of the novel human interferon-alphaWA gene with human interferon-alphaAL genes" & J. Clin. Hematol. Oncol. 1985, 15(1), 1-8

Proc. Natl. Acad. Sci. USA, vol. 81, October 1984, pages 6451-6455, US. R.M. Torczynski et al "Human genomic library screened with 17-base oligonucleotide probes yields a novel interferon gene"

# EP 0 154 186 B1

**Description**

TECHNICAL FIELD

This invention relates to methods and compositions useful in the rapid screening and isolation of genes from genomic libraries. The methods and compositions of the present invention employ small DNA probes, approximately seventeen bases in length, comprising sequences complementary to the gene sought to be screened for or isolated from said genomic library. In one aspect of the present invention, a novel human interferon gene is identified and isolated from a genomic library by methods utilizing small DNA fragments containing nucleotide sequences complementary to at least four known human interferon genes. In another aspect of the present invention, compositions and methods employing recombinant DNA techniques are provided for the replication of the novel human interferon gene in microorganisms.

BACKGROUND ART

Interferons are proteins with tremendous clinical importance and potential. Although a great deal of research has been directed toward an understanding of the molecular mechanism of interferon induction and activity, progress had been slow due largely to the small amounts of pure interferon protein available for study. Recombinant DNA technology offers an opportunity to produce a large amount of both the interferon proteins and the interferon gene sequences. Recombinant DNA technology, however, requires isolation of interferon DNA sequences which is a formidable task since it typically means preparing DNA recombinants from cellular messenger RNA (mRNA) which is present at very low levels and is further complicated by the existence of an as yet unknown number of heterologous interferon proteins and, hence, mRNA molecules. In humans, for example, at least three distinct classes of interferons have been identified, fibroblast, leukocyte and immune interferon. Each class in turn contains several distinct interferon protein species now known to be structurally related within a given class and structurally distinct as between classes.

At the present time, there are two alternative sources from which human interferon genes may be isolated for use in recombinant DNA technology, cDNA libraries prepared from mRNA isolated from human leukocytes synthesizing interferon and genomic libraries prepared from human chromosomal DNA. These libraries are created by inserting either enzymatically synthesized DNA or native human DNA fragments which contain interferon gene sequences into an appropriate vector such as a bacteriophage or plasmid. The insertion of human DNA sequences into such a vector results in the formation of a recombinant or chimeric vector. In recent years, several general methods have been developed which enable construction of such recombinant vectors. For example, U.S. Patent No. 4,237,224 to Cohen and Boyer describes production of recombinant vectors employing restriction endonuclease enzymes and methods of ligation. U.S. Pat. No. 4,237,224 is hereby incorporated by reference into the present specification.

The recombinant vector so produced is then used to transform or infect cells in which the vector is compatible resulting in the introduction of the foreign DNA or genes into the cell. The recombinant vector must be capable of autonomous replication in the host cell and should contain at least one selective marker, a limited number of known restriction endonuclease cleavage sites and a high copy number. The resultant replication of the recombinant vector allows for the generation of multiple identical copies or cloning of said recombinant vector and, hence, the foreign genes inserted into said vector. Each clone or replicated recombinant vector contains a unique fragment of foreign DNA or foreign gene. The term "library" refers to the generation of multiple, distinct recombinant vectors from which a single, specific gene sequence may be selected.

To date, human interferon genes have been isolated from cDNA libraries employing a heterogeneous mixture of cDNA probes containing interferon-specific DNA sequences and from genomic libraries employing similar cDNA probes. Pestka, Archives of Biochem. and Biophys. 221:1 (1983). The use of cDNA libraries for the isolation of interferon genes provides several advantages as well as disadvantages when compared to genomic libraries containing chromosomal DNA. The major advantage of cDNA libraries is their specificity. When prepared from mRNA coding for a desired gene product, a cDNA library should primarily contain only those DNA sequences corresponding to the desired gene product whereas genomic libraries will contain the desired gene sequences along with all the genes and regulatory sequences necessary for the functioning of the entire organism from which the chromosomal DNA was isolated. The disadvantages of cDNA libraries, however, stem from this very specificity and from the mode of construction.

Typically, cDNA libraries are constructed from interferon-specific mRNA isolated from cells producing interferon. This mRNA is present at very low levels in these cells and represents only those interferon genes actively expressed. Interferon genes which are not actively expressed are not retrievable by this method. Additionally, due to the normal cell processes of generating interferon-specific message, the resultant mRNA molecules may no longer contain as a complete complement of nucleotide sequences as compared to the native genes from which the mRNA is generated. Of the eukaryotic genes isolated to date, many of these genes carry sequences, called "introns", within their respective gene sequences. These introns are excised during mRNA transcription and processing so that the resultant mRNA molecules no longer contain the introns. A further loss of gene sequences may occur during the isolation of these very labile and nuclease-sensitive mRNA molecules and employment in the synthesis of cDNA molecules to be contained within the cDNA libraries. The need to transcribe the mRNA molecules into cDNA molecules and then double-stranded DNA which is inserted into the library may result in copying errors and thus possible alterations in the structure and function of the final interferon gene products. Furthermore, construction of both cDNA and genomic libraries may suffer losses in gene sequences during the insertion of the gene sequences into the vector employed to carry the gene library.

Alternatively, genomic libraries contain cloned DNA isolated from cell genomes. Given enough clones, one can generate a library with a 99% chance of containing any gene. Additionally, these cloned genes will be present in their native and genetically complete form along with regulatory signals which may prove advantageous in eukaryotic, as opposed to prokaryotic, cloning systems. Since genomic libraries are generated from native, chromosomal DNA, no further copying is required prior to their insertion into the chosen vector. Furthermore, these inserted double stranded DNA fragments are relatively non-labile and nuclease resistant. Hence, the preferred source for genes not actively expressed, expressed in a low copy number, genes for which there exists an uncertainty regarding its site of synthesis, or genes present in multiple copies or forms is a genomic library.

Genomic libraries are prepared by mechanically shearing chromosomal DNA or cleaving said DNA with restriction endonucleases into 16-20 kilobase (kb) fragments and thereafter inserting said DNA fragments into an appropriate DNA vector as described in "The Isolation of Structural Genes from Libraries of Eucaryotic DNA", Cell 15:687 (1978), Maniatis et al.

One of the major tasks in isolating the desired genes from genomic libraries involves the screening of these libraries for said gene or genes.

A preliminary screening method useful in identifying and enriching for those vectors containing foreign DNA sequences or genes employs "insertional inactivation". This method is described by Bochner et al., Journal of Bacteriology, 143:926 (1980) and comprises insertion of the foreign DNA or genes into a preselected site in a plasmid vector, such insertion resulting in the inactivation of a preselected plasmid gene. Recombinant plasmid vectors can then be identified by screening the plasmids for loss of the specific gene function, in this case, loss of previous antibiotic resistance. An alternative method for enrichment of clones containing recombinant plasmid vectors involves treatment of plasmid with alkaline phosphatase before ligation with the foreign DNA to be cloned as described by Seeburg et al., Nature 220:486 (1977) and Ullrigh et al., Science 196:1313 (1977). These methods, however, do not allow for the identification, localization or isolation of a specifically desired gene or DNA fragment.

The identification and isolation of clones containing specific genes presently requires the use of gene sequence specific probes. One such technique involves the screening of bacterial clones described by Grunstein and Hogness, Proceedings of the National Academy of Science, 79:1844 (1982) or bacteriophage plaques, described by Benton and Davis, Science 196:180 (1977), with labeled probes consisting of natural DNA fragments, synthetic DNA fragments based on known protein sequence information, or gene-specific mRNA molecules utilizing differential hybridization strategies. The employment of gene-specific mRNA molecules presents problems in mRNA isolation as discussed above. Similarly, absent the prior cloning of the natural gene or DNA sequences, the employment of natural DNA fragments as probes is not feasible.

The use of synthetic DNA fragments based on known protein sequence information requires the isolation and sequencing of the gene-specific protein product. Once the protein has been isolated and sequenced, a given region of the protein is selected to provide the gene sequence which is then synthesized by the phosphotriester method described by Agarwal et al., Agnew. Chem. Int. Ed. Engl. 11:451 (1972) or the phosphodiester method described by Itakura et al., J. Biol. Chem. 250:4592 (1975). Coupling of the phosphotriester method with solid-phase synthesis has resulted in the synthesis of entire genes such as alpha interferon as described by Edge et al., Nature 292:756 (1981). Synthesis of these probes is a timely process. Furthermore, due to the degeneracy of the genetic code, mixed-sequence oligodeoxynucleotide probes have been used to isolate protein genes of unknown sequence from cDNA libraries. Suggs et al. Proc. Nat'l. Acad. Sci. U.S.A. 78:6613. Such probes are typically mixtures of oligonucleotides representing every possible codon combination for a given stretch of amino acid sequence. Although these probes, employed in cDNA library screening only, are typically 11-17 nucleotides in length, Singer-Sam et al. Proc. Nat'l. Acad. Sci. U.S.A. 80:802 (1983), the shortness and heterogeneity of these mixed probes often lack the specificity required for probing sequences as complex as a mammalian genome contained within a genomic library.

To date, identification and isolation of mammalian genes from genomic libraries has required the use of synthetic DNA probes of greater than 80 nucleotides in length. Anderson et al. Proc. Nat'l Acad. Sci. U.S.A. 80:6838 (1983). The generation of these relatively long DNA probes involves the synthesis of several short, 12 to 20 nucleotides, oligonucleotides and thereafter linking said oligonucleotides together to yield the final, greater than 80 nucleotides, DNA molecule employed as a probe. Construction of said probes is, again, a timely and tedious process. It is, therefore, desirable to develop methods whereby homogeneous, short, 10-20 nucleotides, DNA sequences may be employed as probes to screen genomic libraries comprising mammalian genes.

Once the gene is identified within the genomic library, the bacteriophage or plasmid, recombinant vector, containing the gene is isolated and replicated by standard methodologies to generate multiple copies of the recombinant vector.

## SUMMARY OF THE INVENTION

The present invention provides methods and compositions for the identification, isolation, cloning of a novel, mature human interferon gene.

In another aspect of the present invention, methods and compositions are provided which allow for the rapid screening and isolation of mammalian genes from genomic libraries. The method of the present invention comprises synthesis of small DNA probes, approximately seventeen nucleotides in length, comprising sequences complementary to the gene or genes sought to be identified and isolated, labeling said DNA probes, thereafter hybridizing the labeled DNA probes to the genomic library employing an improved method for hybridizing the labeled DNA probes to recombinant vectors contained within a host cell, the improvement comprising filtration of the labeled DNA probes prior to their addition to the hybridization solution, employing said labeled DNA probes in the hybridization reaction, employing a device to simultaneously rotate and incubate the nitrocellulose filters during prehybridization and hybridization and adding an effective amount of dextran sulfate to the prehybridization and hybridization solutions, and thereafter identifying said gene in a manner consistent with the genomic library and label chosen. In one example of the present invention, at least two small, labeled DNA probes, approximately 17 nucleotides in length, are synthesized to contain distinct complementary sequences present in at least four of the eight described cDNA alpha-interferon genes. These DNA probes are then employed to screen human genomic libraries for human alpha-interferon genes.

In a further aspect of the present invention, an improved method for hybridizing radiolabeled DNA probes to recombinant vectors contained within a host cell and affixed to nitrocellulose filters, the improvement comprising employment of at least one radiolabeled DNA probe of at least 17 bases in length, filtration of the labeled probe before addition to the hybridization solution, use of a device to simultaneously rotate and incubate filters during prehybridization and hybridization and addition of ten percent dextran sulfate to the prehybridization and hybridization solution.

The methods and compositions of the present invention are particularly useful in the isolation of mammalian genes present in low copy number from genomic libraries and subsequent generation of large quantities of said genes and gene products for clinical diagnosis and therapy.

## BRIEF DESCRIPTION OF THE DRAWINGS

FIG. 1 is a perspective view of the combined incubation and rotational device with a break-away view of the motor and right angle bevel gears.

FIG. 2 is an enlarged fragmentary cross-sectional view of FIG. 1 taken along lines 2-2.

FIG. 3 is a perspective view of the platform unto which the containers for hybridization reaction mixtures are fastened.

FIG. 4 shows the complete base sequence for the human alpha-interferon IFWA gene carried on the vector M13 mp8 along with the corresponding amino acid sequence for the protein for which it codes. "$S_1$" denotes the first of 23 amino acids of the interferon signal peptide. "1" denotes the start of the mature interferon protein.

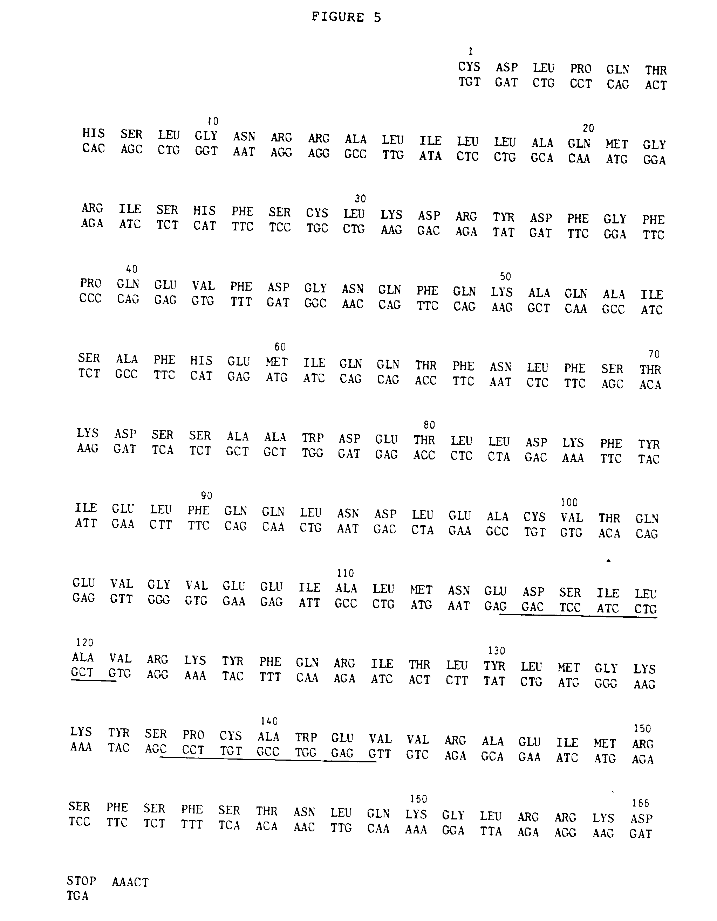

FIG. 5 shows the complete gene sequence of the mature human alpha-interferon WA gene.

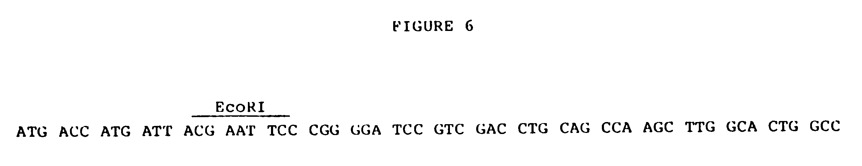

FIG. 6 shows the restriction endonuclease cleavage sites present on the vector M13 mp8 employed in the insertion of human alpha-interferon genes IFWA and IF77 into said vector and restriction endonuclease cleavage sites useful in determining the insertion of said genes into said vector.

DETAILED DESCRIPTION OF THE INVENTION

The present invention relates to methods and compositions which allow for the rapid screening and isolation of known and novel mammalian genes from genomic libraries. Unlike previously described methods for isolating genes from genomic libraries which required the synthesis and employment of large, greater than 80 nucleotides, DNA probes or a mixture of heterogeneous probes identifying a single region of the gene sought to be identified and isolated, the method of the present invention employs small, approximately 17 nucleotides, DNA probes containing sequences complementary to distinct regions on the gene or genes to be identified and isolated.

The employment of small DNA probes, as described in the method of the present invention, provides several advantages over previously described methods for screening and isolating mammalian genes from genomic libraries. The smaller DNA probes are easily and relatively rapidly synthesized by conventional DNA synthetic techniques, allow for selection of gene regions possessing high G:C content which results in a tighter and hence more efficient binding in conventional hybridization screening processes, and allows for the independent identification of desired genes when coupled to duplicate plating procedures which eliminates the high rate of false positive gene identification in conventional hybridization screening processes.

Synthesis of Small DNA Probes

In one example of the present invention, two probes for human alpha-interferon were synthesized, one by Bio Logicals, Inc. (Toronto, Canada) and one using a Bachem Gene Machine obtained from Bachemgentec, Inc. (Torrance, California). The 17-base (17-mer) sequences of each probe were based on sequence information of eight cDNA interferon clones Goeddel et al., Nature 290:20 (1981). Alternatively, sequence information useful for the generation of small DNA probes may be obtained from gene-specific mRNA. Probes based on mRNA sequences may then be synthesized utilizing the enzyme reverse transcriptase which makes cDNA copies of the mRNA Maniatis et al. Molecular Cloning, A Laboratory Manual Cold Spring Harbor Laboratory Publications, New York, New York (1982). For probe A, complementary sequences were present in at least four of the eight described cDNA alpha-interferon genes whereas for three of the other genes there was only one base mismatch and two base mismatches for the last gene. For probe B, complementary sequences were present in at least four of the eight described cDNA alpha-interferon genes whereas for the other four genes there was only one mismatch. Alpha-interferon sequences homologous to the two probes were G:C rich and comprised sequences approximately 50 bases apart. The sequences of the probes were:

| A | B |
|---|---|
| 5'CAGCCAGGATGGAGTCC3' | 5'CCTCCCAGGCACAAGGG3' |

Probe A was synthesized by the solid-phase phosphotriester method and purified as described by Itakura et al. J. Biol. Chem. 250:4592 (1975). Probe B was obtained from Bio Logicals, Inc. (Toronto, Canada). The probes were labeled at the 5'-ends with $T_4$-infected E. coli polynucleotide kinase obtained from P.L. Biochemicals and [$\gamma$32p]-ATP, 5000 Curies per millimole, purchased from New England Nuclear (Boston, Mass.) as follows. Thirty nanograms of Probe A or B was suspended in 10 microliters of water to which 10 microliters 5x polynucleotide kinase (PNK) buffer comprising 0.5M Tris-HCl, pH 7.6, 0.1M $MgCl_2$, 50 mM dithiothreitol, 1mM spermidine and 1mM $Na_2$EDTA, 10 microliters [$\gamma^{32}$p]-ATP (0.002 micromoles per milliliter) and 20 microliters of water were added. Thereafter, 1 microliter (5 units per microliter) PNK was added and the mixture incubated at 37°C for 20 minutes. Following said incubation, an additional 1 microliter PNK was added. Alternatively, a nonisotopic label, such as biotin which is usually linked to thymine, may be incorporated into the DNA probes during synthesis. Biotin binds tenaciously with avidin, a 68,000 dalton protein, and the resulting complex is detectable by a number of biochemical means as described by Brigati et al. Virology 126:32 (1983) and by Leary et al. Proc. Nat'l. Acad. Sci. U.S.A. 80:4045 (1983).

The minimum length of a DNA probe employed to identify and locate a given gene sequence or fragment or allele thereof within a DNA library is determined by both the binding energy of the probe and signal of the label attached thereto used to detect the formation of a stable hybrid. To date, under present conditions employed in hybridization procedures, at least eleven nucleotides are required to establish a stable hybrid. In the method of the present invention, the preferred length of a DNA probe detectable by a radiolabel incorporated into said probe is at least about seventeen nucleotides in length.

The end-labeled DNA probes were then separated from unincorporated $[\gamma\text{-}^{32}P]$-ATP by gel filtration through a Sephadex G-50 column. The columns are typically 30 x 0.6 centimeters and are contained within 2 milliliter disposable pipettes. The columns are equilibrated with a buffer comprising 10mm Tris (tris hydroxymethyl amino methane)-HCl, pH 7.5, and 1mM $Na_2$EDTA (sodium ethylene diaminetetraacetic acid). Approximately 40 fractions comprising 0.05 milliliters per fraction are collected and those fractions containing the first peak of radioactive material eluted from the column are pooled and adjusted to a final buffer concentration 6 x NET which comprises of 0.9M NaCl, 6.0mM $Na_2$EDTA and 0.09M Tris-HCl, pH 7.5, by adding an appropriate amount of 20 x NET. The end-labeled DNA probes are then filtered through a 0.2 micron ACRODISC® filter purchased from Gelman Sciences, Inc. (Ann Arbor, Mich.) which have been pretreated by filtering 50 to 100 micrograms of yeast transfer RNA purchased from Boehringer Mannheim, W. Germany in 10 mM Tris-HCl, pH 7.5, 1.0mM $Na_2$EDTA through the filter. The specific activities of the two probes may range from 1 to 5 x $10^8$ counts per minute (cpm) per microgram of DNA with a preferred specific activity ranging from about 3 to about 5 x $10^8$ cpm per microgram of DNA.

The human genomic library in Lambda Charon 4A, Lawn et al. Cell 15:1157 (1978), obtained from Tom Maniatis (Harvard University, Boston, Mass.), was then screened separately by each probe. E. coli DP.50 (Dr. Edwin C. Murphy, M. D. Anderson Hospital, Houston, TX.) were preinfected with Lambda Charon 4A containing said library, in NZ amine medium prepared as follows. Ten grams NZ amine type A (Humko Sheffield, Memphis, Tenn.), 5 grams yeast extract (DIFCO Laboratories, Detroit, Mich.), 5 grams NaCl, 2 grams $MgCl_2 \cdot 6H_2O$, were dissolved in double distilled water to a final volume of one liter. The medium was then autoclaved at 121°C at 15 pounds per square inches for 25 minutes. Thereafter, filter sterilized thymidine and diaminopimelic acid (DAP) were added to a final concentration of 0.04 grams thymidine and 0.1 grams DAP per liter. Preinfection was performed by incubating said Lambda Charon 4A bacteriophage with the E. coli for 15 minutes at 37°C. The infected E. coli were then plated at a preferred density of 1 x $10^4$ plaque forming units (pfu) per 80 square centimeter dish in NZ amine medium containing 0.7% Sea Kem agarose (FMC, Rockland, Me.) onto prepared plates containing NZ amine medium and 1.2% bactoagar (DiFCO Laboratories, Detroit, Mich.). The cultures were then incubated for 6 hours at 37°C.

Hybridization With Small DNA Probes

A total of about 1.8 x $10^5$ to about 1.9 x $10^5$ pfu were screened using the plaque hybridization method of Benton and Davis, Science 196:180 (1977), which included the following modifications: (1) use of two, sequentially different labeled probes, (2) separation of said probes from contaminating and/or interfering substances prior to their addition to the hybridization mixture, (3) use of a device to rotate filters and control the environmental temperature during prehybridization and hybridization, and (4) use of an effective amount of dextran sulfate in the hybridization solution. Upon screening 1.8 x $10^5$ pfu, one would be 64 percent confident that a given DNA sequence would be represented. Maniatis et al. Molecular Cloning, A Laboratory Manual, Cold Spring Harbor Laboratory Publications New York, New York (1982).

In an alternative embodiment, the plaque hybridization method of Benton and Davis (1977) includes the following modifications: (1) use of one DNA probe approximately 17 nucleotides in length and homologous to a region in the gene, gene fragment or genes, gene fragments to be identified, (2) separation of said probe from contaminating and/or interfering substances prior to its addition to the hybridization mixture, (3) use of a device to rotate filters and control the environmental temperature during prehybridization and hybridization, (4) use of an effective amount of dextran sulfate in the prehybridization and hybridization solution, and (5) a parallel hybridization with said probe on duplicate filters of the plaques being screened.

The separation of the radiolabeled DNA probes from contaminating and/or interfering substances prior to their addition to the hybridization solution may be achieved by, for example, filtration which removes particles having a greater solution diameter than the probes. Such filtration appears to remove substances which otherwise cause non-specific adherence of these probes to the nitrocellulose filters and thus reduces the background found when the probes are not previously filtered. These substances are hereinafter collectively referred to as interfering substances. A preferred method for separating interferring substances from said probes employs an inert filter with a pore size of approximately 0.2 micrometers or microns. An example of one such filter useful in separating DNA probes from such interfering substances is the 0.2 micron ACRODISC® filter made by Gelman Sciences, Inc. (Ann Arbor, Mich.).

7

Dextran sulfate was added to the prehybridization and hybridization solutions to reduce non-specific binding of the DNA probes and remaining interfering substances to the nitrocellulose filters. It is further known that the rate of association of nucleic acids is accelerated in the presence of dextran sulfate because the nucleic acids, herein the DNA probes, are excluded from the volume of solution occupied by the polymer. The effective concentration of the DNA probes is therefore increased. An effective amount of dextran sulfate was added to the hybridization solution to reduce the final solution volume resulting in an increased solution concentration of DNA probe or probes present in the hybridization solution. This increased DNA probe concentration presumably maximizes the contact between the probes and the complementary DNA sequences contained within the DNA on the nitrocellulose filters and thus increases the rate of association. Wahl et al. Proc. Nat'l. Acad. Sci. 76:3683 (1979) has reported that the rate of association increases 10-fold in the presence of 10% dextran sulfate. A preferred amount of dextran sulfate in the prehybridization and hybridization solutions is about 10% (w/v). Although dextran sulfate is useful in circumstances where the rate of hybridization is the limiting factor in detecting sequences of interest, it is difficult to handle because of its viscosity and is considered unnecessary for most purposes. Maniatis et al. Molecular Cloning: A Laboratory Manual, Cold Spring Harbor Laboratory (1982) p. 325. The use of a constant circular rotation during the prehybridization and hybridization reaction is believed to overcome the viscosity problems attendant to use of dextran sulfate and thereby provide a process wherein dextran sulfate may be employed to enhance the detection of gene sequences described in the present invention.

Duplicate nitrocellulose filter copies were made from each plate in accordance with the method described by Benton and Davis, Science 196:180 (1977). The BA85SD nitrocellulose filters were obtained from Schleicher and Schuell, Inc. (Keene, N.H.). The nitrocellulose filter copies were then alkali denatured on 3MM Whatman filter paper, saturated with 0.5N NaOH and 1.5M NaCl for 5 minutes, neutralized on paper saturated with 1M Tris-HCl, pH 7.5, for 5 minutes and again neutralized on paper saturated with 0.5M Tris-HCl, pH 7.5, and 1.5M NaCl for 5 minutes. The nitrocellulose filters were then baked under a vacuum of 20 to 25 inches of mercury in a National Appliance vacuum oven at 80°C for two hours. The nitrocellulose filters were next placed in KAPAK/Scotch PAK™ heat sealable pouches, Fisher Scientific (Pittsburgh, Pa.), to which was added 5 milliliters of prehybridization solution comprising 6 x NET, 0.1% weight per volume (w/v) sodium dodecyl sulfate (SDS) and 10% (w/v) dextran sulfate Sigma Chemical Company (St. Louis, Mo.), and thereafter the bag was sealed with a Scotch PAK® Pouch Sealer, Fisher Scientific (Pittsburgh, Pa.). A 50% (w/v) stock dextran sulfate, Sigma Chemical Company (St. Louis, MO.), solution is prepared in deionized water and stored at minus 20°C.

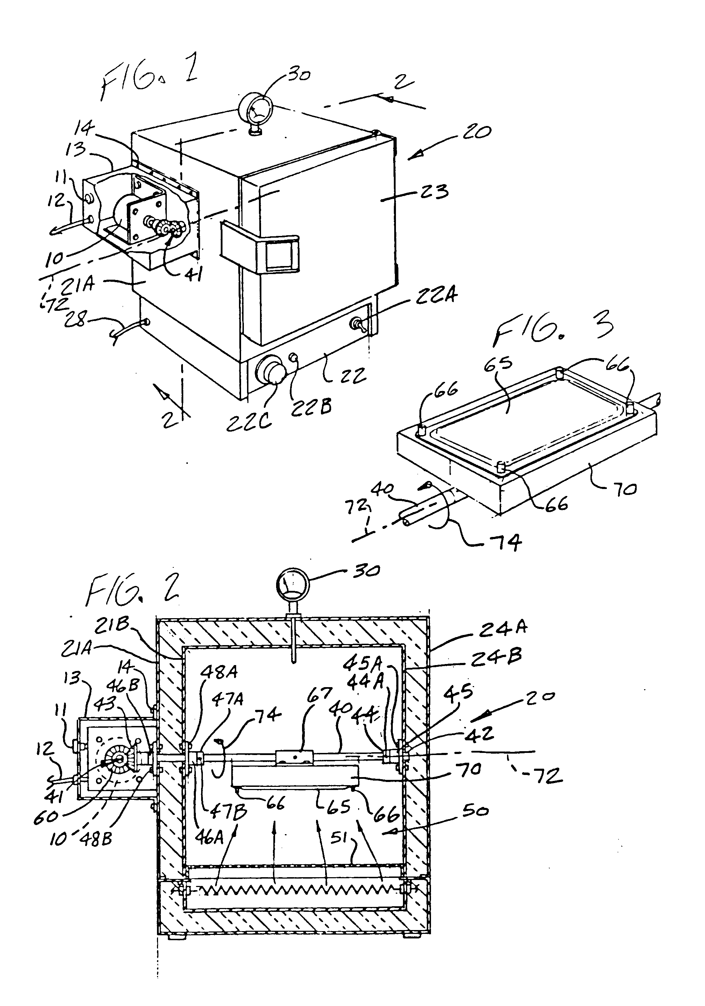

The sealed pouches were placed in a rotation device shown in FIGS. 1-3 which simultaneously rotates the pouches 360° and provides a controlled temperature environment. As shown in FIG. 1, a conventional electric motor 10 with its own "on/off" power switch 11 and power cord 12 and cover 13, is mounted by appropriate fastening means 14 midway on one outside wall 21A of a conventional oven 20, such as a Stable-Therm® Gravity Oven purchased from Fisher Scientific (Pittsburgh, Pa.). Said oven 20 being capable of maintaining a constant temperature with a termperature range of from ambient to about 250°C. A Temperature indicator 30 denotes the temperature inside the oven 20. A front-facing panel 22 located below a front opening door 23 contains a main power "on/off" switch 22A for said oven 20, a temperature indicator "on" light 22B and a temperature selection and control device 22C. The oven 20 has its own power cord 28. As show in in FIG. 2, an axle 40 capable of continuous circular rotation about its central axis 72 and comprising, for example, a metal rod having a first end 41 and second end 42, runs horizontally through the center of the oven chamber 50 so as to run parallel to the floor 51 of the oven chamber 50 and perpendicular to the oven chamber 50 inner side walls 21B and 24B. The position and rotational movement of the axle 40 is maintained by securing the second end 42 to the inner side wall 24B by means of a bearing 45, said bearing 45 being attached to the inner side wall 24B by appropriate fastening means 45A, and by securing the first end 41 comprising driven beveled gear 43 to the inner side wall 21B by means of a bearing 46A, said bearing 46A being attached to the inner side wall by appropriate fastening means 48A, and to the outside wall 21A by means of a bearing 46B, said bearing 46B being attached to the outside wall 21A by appropriate fastenings means 48B, as shown in FIGS. 2 and 3 in the direction of arrow 74. The circular rotation, shown in FIG. 2, of the axle 40 is achieved by meshing the motor 10 powered driving beveled gear 60 with the driven beveled gear 43. The axial movement of the axle 40 along axis 72 is prevented by means of a collar 47A and set screw 47B positioned at the first end 41 of the axle 40 and a collar 44 and set screw 44A positioned at the second end 42 of the axle 40 as shown in FIG. 2. A platform 70 for mounting the sealed hybridization pouches 65 to the axle 40 is shown in FIGS. 2 and 3. The platform 70 comprises any suitable material, such as styrofoam™ or wood, to which the sealed hybridization pouches 65 may be securely mounted by such fastening means 66 as needles, pins, tacks, nails or the like, without puncturing the fluid-filled portion of the sealed hybridization pouches 65. The platform 70 is attached to the axle 40 by a suitable locking means 67 as shown in FIG. 2, for example nuts and bolts and the like so that the platform 70 will circularly rotate in conjunction with the circular rotation of the axle 40. In one process conducted under the teachings of the present invention, the pouches were rotated at 10 revolutions per minute (rpm) at 37°C for about 12 to 24 hours. The rotation of the filters in the device of FIGS. 1-3 optimizes the movement of the prehybridization solution over the nitrocellulose filters. Following the incubation period, a corner of the pouch was cut open and the prehybridization solution was removed by aspiration. Two milliliters of solution, identical to the prehybridization solution, was added plus radiolabeled probe to a concentration of about one to about ten nanograms per milliliter with a preferred probe concentration of about two nanograms per milliliter of solution.

In the modified hybridization procedure employing a single DNA probe, herein either Probe A or Probe B, a single probe is added to the hybridization solutions applied to the duplicate filters. In the present example of the invention, both Probes, A and B, were employed so that only Probe A was added to one filter hybridization reaction and Probe B was added to the duplicate filter hybridization reaction.

Hybridizations were performed at 37°C for about 20 to about 24 hours with constant rotation as described for the prehybridization incubation above. Following the incubation period, the nitrocellulose filters were removed from the bags and placed in a suitable size pyrex™ dish containing 250 milliliters of wash buffer comprising 6X SSC, wherein 1X SSC comprises 0.15M sodium chloride, 0.015M sodium citrate. The filters were washed four times, twenty minutes per wash, in 250 milliliters of wash buffer, at room temperature with gentle rocking and, thereafter, washed four times in 250 milliliters 6X SSC containing 0.1% SDS at 45°C for about one hour. The nitrocellulose filters were then air dried at room temperature for about 30 minutes. Autoradiography was then performed using Kodak™ XAR film plus two intensifying screens, Dupont Cronex Hi Plus™, for about 64 to 72 hours at minus 80°C. The filter and overlying Kodak™ XAR film were sandwiched between the two intensifying screens. Bacteriophage plaques which hybridized to the DNA probe were identified by common autoradiographic signals on duplicate filters hybridized with probes A and B, respectively. By employing and comparing the signals of a single DNA probe or two sequentially different DNA probes in parallel hybridizations conducted on duplicate filters, one may eliminate false positive hybridization signals typically occurring in a single hybridization reaction. The positive plaques were then isolated by several cycles of plaque purification as described by Benton and Davis, Science 196:180 (1977).

Due to the high density bacteriophage plating, approximately 1 x 10⁴ pfu per 80 square centimeter dish, during the initial screening of the Lambda Charon 4A genomic library, the isolated phage which hybridized to both probes, A and B, were subjected to a second infection and plating with E. coli DP. 50 at a lower density of about 500 pfu per 80 square centimeter dish as described for the initial screening. The bacteriophage plaques were then screened in a manner identical to that described above. This second screening yielded three bacteriophage plaques, designated λ77, λWA and λ105 respectively, which hybridized to both probes, A and B. Multiple copies of λ77, λWA and λ105 were generated by the method described by Maniatis et al. Molecular Cloning, A Laboratory Manual, Cold Spring Harbor Laboratories, Cold Spring Harbor, New York, New York (1982).

Localization and Isolation of Specific Genes Contained Within Recombinant Vectors

The plaque-purified clones, IF77, IFWA and IF 105, which hybridized to both probes, A and B, were then subjected to further analysis to localize and isolate the specific gene or genes, in the example herein, human alpha-interferon. Localization of specific genes or gene fragments contained within a recombinant vector is typically conducted by fragmenting the recombinant vector with restriction endonuclease enzymes. Any restriction enzyme or combination thereof may be used. Ideally, the restriction enzymes employed should not destroy the gene or portion thereof sought to be isolated. The restriction enzymes used for gene localization and isolation are therefore selected with the gene location gene sequence, if known, and cloning vector in mind.

Previous studies with alpha-interferon genes isolated from cDNA libraries, Pestka, Archives of Biochem. and Biophys. 221:1 (1983) indicate that alpha-interferon genes are approximately 400 nucleotides in length and do not contain BamHI or EcoRI restriction endonuclease sites within the gene sequences. These restriction enzymes are therefore suitable for use in isolating alpha-interferon genes from the plaque-purified clones. To insure that an entire gene is retrieved from the purified bacteriophage, and to facilitate the subsequent insertion of said gene into cloning and expression vectors, a restriction endonuclease fragment of approximately 1 to 2 kilobases (kb) containing the gene, is desired. These enzymes may also be employed to isolate interferon gene fragments, tandem genes and/or an allele of said gene wherein an allele comprises a gene sequence differing in less than or equal to one percent of a given gene sequence.

In the example of the present invention, clones IF77, IFWA and IF105 were subjected to analysis by restriction endonuclease cleavage with restriction enzymes BamHI, DdeI and EcoRI, purchased from Bethesda Research Laboratories, Inc. (Gaithersburg, Md.) followed by Southern hybridization, Southern J. Mol. Biol. 98:503 (1975), with the two end-labeled alpha-interferon probes, A and B, to identify the restriction endonuclease fragments containing the alpha-interferon gene or portion thereof.

Specifically, bacteriophage DNA from the clones was isolated as described by Maniatis et al. Molecular Cloning, A Laboratory Manual, Cold Spring Harbor Publications, New York, New York (1982) Chapter 3, and individually digested with one of the following restriction endonucleases, BamHI, EcoRI or DdeI, under conditions set forth in Example 1, below. Thereafter, the restricted DNA was fractionated by agarose gel electrophoresis in one percent, weight per volume (w/v), agarose (Sea Kem) purchased from FMC Corp. (Rockland, Me.) in gel buffer comprising 90 mM Tris base, 90mM boric acid and 2.5mM Na$_2$EDTA, and transferred to nitrocellulose as described by Southern, J. Mol. Biol. 98:503 (1975). Probes A and B were then individually hybridized to the restricted DNA-containing nitrocellulose, in a manner identical to that previously described for the initial bacteriophage screening, to identify those fragments containing alpha-interferon gene sequences. Appropriately labeled alpha-interferon specific mRNA or cDNA may alternatively be employed to identify the restriction endonuclease fragments which contain alpha-interferon gene sequences. As shown in Example 1, below, cleavage of clones λ77 and λWA with the restriction enzyme EcoRI yielded DNA fragments approximately 1.8 and 2.0kb, respectively. These EcoRI restriction fragments are sizes suitable for effective gene cloning and subsequent gene expression.

Cloning of Isolated Genes

Once the gene, gene fragment or allele is isolated, the gene is inserted into an appropriate cloning vector which provides a means for replicating the gene. Any appropriate cloning vector containing a marker function may be used, for example, E. coli plasmid vectors which include Col EI, Hershfield et al. Proc. Nat'l. Acad. Sci. U.S.A. 71:3455 (1974), pBR322, Bolivar et al. Gene 2:95 (1977), pBR325, Soberon et al. Gene 4:121 (1978) and pKC7, Rao et al. Gene 7:79 (1979) and E. coli bacteriophage vectors which include Charon λL47.1 Loenen et al. Gene 10:249 (1980), and M13 mp8, Messing et al. Gene 19:269 (1982).

Insertion of the gene, gene fragment or allele into the cloning vector to create a recombinant cloning vector is accomplished by cleaving the cloning vector with restriction enzymes which yields cohesive ends that are complementary to those of the gene, or so modifying these ends to create complementary sequences, and then ligating the two fragments. Absent cohesive ends on the gene or gene fragment or following restriction enzyme cleavage, the gene may be blunt-end ligated to the cleaved cloning vector. Such cloning vectors may also be employed to replicate wholly synthetic genes.

Once ligated, the recombinant cloning vector is used to transform or infect the appropriate host cells. The host cells chosen must allow for the replication of the recombinant cloning vector and, preferably, in high copy number.

In the example of the present invention, M13 mp8, describd by Messing et al. Gene 19:269 (1982), was chosen as the cloning vector. The M13 mp8 vector allows for isolation of recombinant vectors in both double stranded (ds) DNA and single stranded (ss) DNA forms. Isolation of ds DNA recombinant vectors facilitates subsequent cloning in expression vectors, described below, and isolation of ss DNA recombinant vectors facilitates isolation of recombinant vectors which contain the gene in a proper 5' to 3' orientation for gene expression. Additionally, M13 mp8 can accommodate genes or gene fragments ranging from 1 to 4kb in length which insures the cloning of an entire gene sequence.

In the example of the present invention, the 2kb and 1.8Kb EcoRI fragments from bacteriophage clones λWA and λ77, respectively, were inserted into the unique EcoRI site on the M13 mp8 vector to create recombinant cloning vectors by the method described by Messing et al. Gene 19:269 (1982). The EcoRI fragments isolated from clones λWA and λ77 are hereinafter referred to as alpha-interferon genes IFWA and IF77, respectively. Insertion of IFWA or IF77 into the M13 mp8 cloning vector was accomplished as follows. Approximately 1.0 micrograms of M13 mp8 DNA, was diluted in 100 microliters of digestion buffer comprising 50 mM Tris-HCl, pH 7.5, 0.01M MgCl$_2$, 50 mM NaCl, 1mM dithiothreitol and 100 micrograms bovine serum albumin to which was added 0.5 to 1.0 units of EcoRI (Bethesda Research Laboratories, Inc., Gaithersburg, Md.). The mixture was incubated 60 minutes at 37° C and then treated with 1 unit of calf intestine alkaline phosphatase (Boehringer-Mannheim, W. Germany) at 37° C for 30 minutes. Thereafter, the mixture was phenol extracted two times to remove the above enzymes and thereafter ether extracted one time to remove residual phenol and then ethanol precipitated. Ten micrograms of IFWA and IF77 was digested with EcoRI in the same manner as M13 and mp8 digestion with EcoRI. The digested Mp13 mp8, IFWA and IF77 DNA's were then individually electrophoresed on 1% low melting temperature agarose gels.

The 2kb EcoRI restriction fragment of IFWA and the 1.8 kb fragment of IF77, each containing approximately 400 nanograms of DNA were isolated and purified from the agarose as described by Maniatis et al. Molecular Cloning, A Laboratory Manual, Cold Spring Harbor Publications, New York, New York (1982). Thereafter, the digested M13 mp8 DNA was mixed with each respective EcoRI restriction fragment in 20 microliters of ligation buffer comprising 0.066 M Tris-HCl, pH 7.6, 5.0mM MgCl$_2$, 5.0mM dithiothreitol and 1.0mM ATP, to which 2 units of T$_4$ induced DNA ligase (Bethesda Research Laboratories, Inc., Gaithersburg, Md.) was added. The reaction mixture was incubated at 12°C for 16 hours. The recombinant cloning vectors comprising M13 mp8-WA or M13 mp8-77 thus formed were then used to transfect E. coli JM103 obtained from Bethesda Research Laboratories, Inc. (Gaithersburg, Md.).

Transfection of E. coli JM103 grown to an O.D. of 0.6 in 2 x YT medium comprising 20 grams bactotryptone, 10 grams Bacto yeast extract, 5 grams NaCl dissolved in water to a final volume of one liter and sterilized by autoclaving as previously described, was performed by the method described by Messing, Methods in Enzymology 101:20 (1983). Briefly, JM103 cells were pelleted by centrifugation and resuspended in ice cold 50mM CaCl$_2$ and thereafter incubated for 20 minutes on ice. The JM103 cells were then again pelleted by centrifugation and resuspended in one tenth the original cell culture volume in ice cold 50mM CaCl$_2$ and then placed on ice. Approximately one to ten nanograms of recombinant cloning vector DNA was then added to the CaCl$_2$ treated JM103 cells and the mixture incubated on ice for 60 minutes. The transfected cells were heat shocked at 42°C for 2 minutes, then added to 2.5 ml of 2 x YT top agarose comprising 2 x YT medium containing 0.7% Sea Kem agarose and 10 microliters of a 100 mM isopropyl thiogalactopyramoside (IPTG) stock. (Sigma Chemical Co., St. Louis, Mo.), and 50 microliters of 2% (w/v) 5-Bromo-4-Chloro-3 indoyl B-D-galactoside (x-gal) dissolved in dimethyl formamide. The transfected cells were then mixed with 0.1 milliliters of log phase culture JM103 cells. The mixture was then agitated and then poured on agar plates composed of 2 x YT medium containing 1.2% bacto-agar. The plates were incubated at 37°C for 12 to 24 hours.

Approximately 100 to 500 M13 bacteriophage plaques were then isolated. The bacteriophage DNA from each plaque isolated was then individually screened for the presence of alpha-interferon specific DNA by hybridization with one of the end-labeled probes, probe A, as described for the initial bacteriophage screening. M13 mp8 bacteriophage DNA which hybridized to probe A was isolated, as described above, and subjected to restriction endonuclease cleavage with EcoRI to confirm the insertion of IFWA and IF77 into the M13 mp8 vector. The positive hybridization of probe A to the recombinant M13 mp8 vector DNA identifies only those recombinant vectors which contain alpha-interferon gene sequences, IFWA or IF77, in the proper orientation.

To further confirm the isolation of interferon-specific gene sequences and to facilitate the subsequent insertion of said gene sequences into cloning and expression vectors, DNA sequence analysis of the entire isolated gene or gene fragment, was performed by sequencing the individual restriction endonuclease fragments generated from the isolated clones by the DNA sequencing method of Maxam and Gilbert, Methods in Enzymology 36:271(1980). DNA sequencing of the entire ssDNA of the recombinant cloning vector was performed by the Sanger et al. Proc. Nat'l. Acad. Sci. U.S.A., 74:5463 (1977) dideoxy sequencing system utilizing the interferon probes A, B or selected restriction fragments as primers for the DNA sequencing reactions.

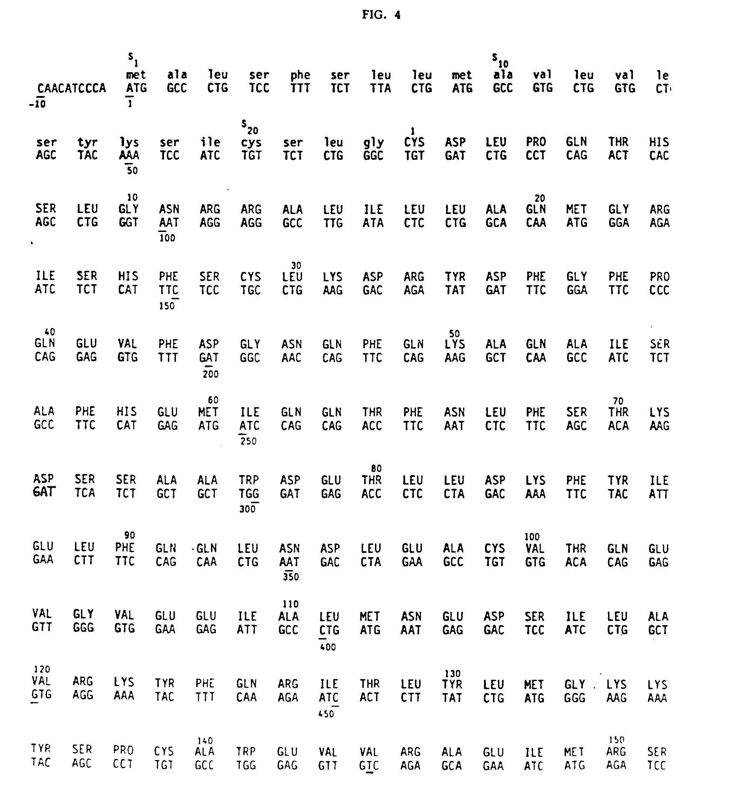

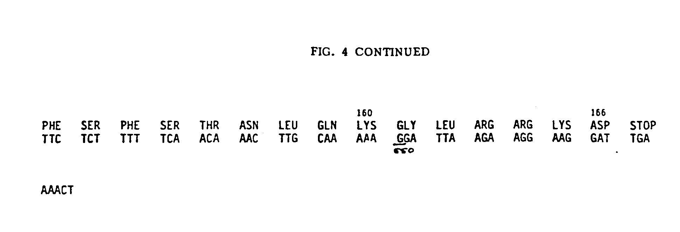

The partial DNA sequencing of IF77, approximately 80% of the gene sequence, strongly suggests that IF77 is identical to human alpha-interferon L isolated from a human cDNA library as described by Pestka, Archives of Biochem. and Biophys. 221:1 (1983). The complete DNA sequence of IFWA, shown in FIG. 4, however, displays a human alpha-interferon gene sequence heretofore not disclosed in the available scientific literature and is therefore determined to represent a novel human alpha-interferon gene. The complete IFWA gene sequence shown in FIG. 4 presents both the signal peptide (s) and mature protein (m) sequences. The signal peptide gene product guides the secretion of the mature interferon protein from the interferon producing cell. The isolation of a known human alpha-interferon gene, IF77, attests to the efficacy of the methods and compositions of the present invention, as set forth herein, in the isolation of such genes as alpha-interferon from genomic libraries. Furthermore, the isolation a novel human alpha-interferon gene, IFWA, not previously identified or isolated by prior techniques, is possibly indicative of the sensitivity of the methods and compositions of the present invention.

## Claims

1. A method for identifying a gene, gene fragment or allele contained within a genomic or cDNA library in the form of recombinant vectors contained within a host cell by
   (a) synthesizing at least one unique sequence DNA probe comprising a DNA sequence homologous to at least one DNA sequence contained within said gene, gene fragment or allele thereof and of an effective size to stably hybridize to said gene, gene fragment or allele,

(b) labeling said DNA probe,

(c) hybridizing said labeled DNA probe to said genomic or cDNA library affixed to nitrocellulose filters and

(d) thereafter identifying said gene or gene fragment in a manner consistent with the label chosen, the improvement comprising:

(i) separating the labeled DNA probe from particulate matter having a solution diameter greater than said labeled DNA probe prior to the addition of said labeled DNA probe to a hybridization solution,

(ii) adding said labeled DNA probe to the hybridization solution containing an effective amount of dextran sulfate capable of increasing the concentration of the DNA probe in the hybridization solution, and

(iii) employing a device to simultaneously rotate and incubate the nitrocellulose filters during prehybridization and hybridization.

2. The method of claim 1, wherein said DNA probe comprises a 17-mer with a base sequence of:
5'CAGCCAGGATGGAGTCC3'

3. The method of claims 1 or 2, wherein said DNA probe comprises a 17-mer with a base sequence of:
5'CCTCCCAGGCACAAGGG3'

4. The method of claim 1, wherein said device is capable of rotating said nitrocellulose filters 360° in a continuous fashion.

5. The method of claims 1 to 4, wherein said labeled DNA probe is separated from particulate matter by passing said labeled DNA probe through an inert filter means providing a means for retaining particles in excess of about 0.2 micrometers.

6. The method of claims 1 to 5, wherein said effective amount of dextran sulfate is about 10 percent (w/v).

7. The method of claims 1 to 6, wherein said prehybridization and/or hybridization solution comprises 0.9M NaCl, 6.0mM $Na_2$EDTA, 0.9M Tris-HCl, pH 7.5, 0.1% (w/v) SDS and 10% (w/v) dextran sulfate.

8. The method of claims 1 to 6, wherein said hybridization solution comprises 0.9M NaCl, 6.0mM $Na_2$EDTA, 0.09M Tris-HCl, pH 7.5, 0.1% (w/v) SDS, 10% (w/v) dextran sulfate and said filtered, labeled DNA probe.

9. The method of claims 1 to 8, wherein two labeled distinct DNA probes, approximately 17 nucleotides in length are used.

10. The method of claim 9, wherein the DNA probes according to claims 2 and 3 are used for the identification of a human interferon gene.

11. A seventeen base DNA fragment of the sequence:
5'CAGCCAGGATGGAGTCC3'

**Revendications**

1. Méthode d'identification d'un gène, fragment de gène ou allèle contenus dans une banque d'ADN génomique ou d'ADNc sous la forme de vecteurs recombinants contenus dans une cellule hôte par

(a) synthèse d'au moins une sonde d'ADN à séquence unique comprenant une séquence d'ADN homologue d'au moins une séquence d'ADN contenue dans ledit gène, fragment de gène ou allèle de celui-ci et ayant une taille efficace pour s'hybrider de façon stable audit gène, fragment de gène ou allèle,

(b) marquage de ladite sonde d'ADN,

(c) hybridation de ladite sonde d'ADN marquée à ladite banque d'ADN génomique ou d'ADN c, fixée sur des filtres de nitrocellulose et

(d) identification dudit gène ou fragment de gène d'une manière compatible avec le marquage choisi, dans laquelle l'amélioration consiste à :

(i) séparer la sonde d'ADN marquée d'une matière particulaire ayant un diamètre en solution supérieur à ladite sonde d'ADN marquée avant l'addition de ladite sonde d'ADN marquée à une solution d'hybridation,

(ii) ajouter ladite sonde d'ADN marquée à ladite solution d'hybridation contenant une quantité efficace de sulfate de dextrane capable d'augmenter la concentration de la sonde d'ADN dans la solution d'hybridation, et

(iii) employer un dispositif pour simultanément mettre en rotation et incuber les filtres de nitrocellulose pendant la préhybridation et l'hybridation.

2. Méthode selon la revendication 1, dans laquelle ladite sonde d'ADN comprend un oligomère à 17 unités ayant une séquence de bases :

5'CAGCCAGGATGGAGTCC3'

3. Méthode selon la revendication 1 ou 2, dans laquelle ladite sonde d'ADN comprend un oligomère de 17 unités ayant une séquence de bases :

5'CCTCCCAGGCACAAGGG3'

4. Méthode selon la revendication 1, dans laquelle ledit dispositif est capable de mettre en rotation lesdits filtres de nitrocellulose sur 360° d'une manière continue.

5. Méthode selon l'une des revendications 1 à 4, dans laquelle ladite sonde d'ADN marquée est séparée de la matière particulaire par passage de ladite sonde d'ADN marquée à travers un filtre inerte fournissant un moyen destiné à retenir les particules supérieures à 0,2 micromètres.

6. Méthode selon l'une des revendications 1 à 5, dans laquelle ladite quantité efficace de sulfate de dextrane est d'environ 10% (p/v).

7. Méthode selon l'une des revendications 1 à 6, dans laquelle ladite solution de préhybridation et/ou d'hybridation comprend 0,9M Nacl, 6,0mM $Na_2$EDTA, 0,9M Tris-HCl, pH 7,5, 0,1% (p/v) SDS et 10% (p/v) sulfate de dextrane.

8. Méthode selon l'une des revendications 1 à 6, dans laquelle ladite solution d'hybridation comprend 0,9M NaCl, 6,0mM $Na_2$EDTA, 0,09M Tris-HCL, pH 7,5, 0,1% (p/v) SDS, 10% (p/v) sulfate de dextrane et ladite sonde d'ADN marquée et filtrée.

9. Méthode selon l'une des revendications 1 à 8, dans laquelle on utilise deux sondes d'ADN marquées distinctes d'environ 17 nucléotides de longueur.

10. Méthode selon la revendication 9, dans laquelle lesdites sondes d'ADN selon les revendications 2 et 3 sont utilisées pour identifier un gène d'interféron humain.

11. Fragment d'ADN de 17 bases de séquence :

5'CAGCCAGGATGGAGTCC3'

**Patentansprüche**

1. Verfahren zur Identifizierung eines Gens, Genfragments oder Allels, das in einer genomischen oder cDNA-Bibliothek in Form von rekombinanten Vektoren enthalten ist, die in einer Wirtszelle enthalten sind, durch

(a) Synthese von mindestens einer DNA-Sonde mit einmaliger Sequenz, die eine DNA-Sequenz enthält, die zu mindestens einer DNA-Sequenz homolog ist, die in besagtem Gen, Genfragment oder Allel davon enthalten ist, und von einer wirksamen Größe, um stabil mit dem besagten Gen, Genfragment oder Allel zu hybridisieren,

(b) Markierung der besagten DNA-Sonde,

(c) Hybridisierung der besagten markierten DNA-Sonde mit der besagten genomischen oder cDNA-Bibliothek, die an Nitrocellulosefilter fixiert ist und

(d) danach Identifizierung des besagten Gens oder Genfragments in einer mit der gewählten Markierung im Einklang stehenden Weise, wobei die Verbesserung umfaßt:

(i) Trennung der markierten DNA-Sonde von partikulären Stoffen, die einen Lösungsdurchmesser größer als die besagte markierte DNA-Sonde haben, vor der Zugabe von der besagten markierten DNA-probe zu einer Hybridisierungslösung,

(ii) Zugabe der markierten DNA-Probe zu der Hybridisierungslösung, die eine wirksame Menge Dextransulfat enthält, welche im Stande ist, die Konzentration der DNA-Sonde in der Hybridisierungslösung zu erhöhen, und

(iii) Verwendung einer Vorrichtung, um die Nitrocellulosefilter während der Prähybridisierung und Hybridisierung gleichzeitig zu rotieren und inkubieren.

2. Verfahren nach Anspruch 1, worin die besagte DNA-Probe ein 17-mer mit der Basensequenz:

5'CAGCCAGGATGGAGTCC3'

enthält.

3. Verfahren nach den Ansprüchen 1 oder 2, worin die besagte DNA-Probe ein 17-mer mit der Basensequenz:

5'CCTCCCAGGCACAAGGG3'

enthält.

4. Verfahren nach Anspruch 1, worin die besagte Vorrichtung im Stande ist, die besagten Nitrocellulosefilter kontinuierlich um 360° zu rotieren.

5. Verfahren nach den Ansprüchen 1 bis 4, worin die besagte markierte DNA-Sonde von partikulären Stoffen durch Passieren der besagten markierten DNA-Sonde durch ein inertes Filtermittel getrennt wird, wobei ein Mittel zur Zurückhaltung von Partikeln größer als 0,2 $\mu$m bereitgestellt wird.

6. Verfahren nach den Ansprüchen 1 bis 5, worin die besagte wirksame Menge an Dextransulfat etwa 10% (g/v) beträgt.

7. Verfahren nach den Ansprüchen 1 bis 6, worin die besagte Prähybridisierungs- und/oder Hybridisierungslösung 0,9 M NaCl, 6,0 mM Na$_2$EDTA, 0,9 M Tris-HCL, pH 7,5, 0,1% (g/v) SDS und 10% (g/v) Dextransulfat enthält.

8. Verfahren nach den Ansprüchen 1 bis 6, worin die besagte Hybridisierungslösung 0,9 M NaCl, 6,0 mM Na$_2$EDTA, 0,09 M Tris-HCl, pH 7,5, 0,1% (g/v) SDS, 10% (g/v) Dextransulfat und die besagte filtrierte, markierte DNA-Sonde enthält.

9. Verfahren nach den Ansprüchen 1 bis 8, worin zwei markierte unterschiedliche DNA-Sonden mit einer Länge von ungefähr 17 Nukleotiden verwendet werden.

10. Verfahren nach Anspruch 9, worin die DNA-Sonden gemäß Anspruch 2 und 3 zur Identifizierung eines humanen Interferongens verwendet werden.

11. Ein 17-Basen DNA-Fragment mit der Sequenz:

5'CAGCCAGGATGGAGTCC3'.

EP 0 154 186 B1

FIG. 1

FIG. 3

FIG. 2

FIG. 4

| | | S₁ | | | | | | | | | S₁₀ | | | | |
|---|---|---|---|---|---|---|---|---|---|---|---|---|---|---|---|
| | | met | ala | leu | ser | phe | ser | leu | leu | met | ala | val | leu | val | le |
| CAACATCCCA | | ATG | GCC | CTG | TCC | TTT | TCT | TTA | CTG | ATG | GCC | GTG | CTG | GTG | CT( |
| -10 | | 1 | | | | | | | | | | | | | |

| | | | | | S₂₀ | | | | 1 | | | | | | |
|---|---|---|---|---|---|---|---|---|---|---|---|---|---|---|---|
| ser | tyr | lys | ser | ile | cys | ser | leu | gly | CYS | ASP | LEU | PRO | GLN | THR | HIS |
| AGC | TAC | AAA | TCC | ATC | TGT | TCT | CTG | GGC | TGT | GAT | CTG | CCT | CAG | ACT | CAC |
| | | 50 | | | | | | | | | | | | | |

| | | 10 | | | | | | | | | | 20 | | | |
|---|---|---|---|---|---|---|---|---|---|---|---|---|---|---|---|
| SER | LEU | GLY | ASN | ARG | ARG | ALA | LEU | ILE | LEU | LEU | ALA | GLN | MET | GLY | ARG |
| AGC | CTG | GGT | AAT | AGG | AGG | GCC | TTG | ATA | CTC | CTG | GCA | CAA | ATG | GGA | AGA |
| | | | 100 | | | | | | | | | | | | |

| ILE | SER | HIS | PHE | SER | CYS | 30 LEU | LYS | ASP | ARG | TYR | ASP | PHE | GLY | PHE | PRO |
|---|---|---|---|---|---|---|---|---|---|---|---|---|---|---|---|
| ATC | TCT | CAT | TTC | TCC | TGC | CTG | AAG | GAC | AGA | TAT | GAT | TTC | GGA | TTC | CCC |
| | | | 150 | | | | | | | | | | | | |

| 40 GLN | GLU | VAL | PHE | ASP | GLY | ASN | GLN | PHE | GLN | 50 LYS | ALA | GLN | ALA | ILE | SER |
|---|---|---|---|---|---|---|---|---|---|---|---|---|---|---|---|
| CAG | GAG | GTG | TTT | GAT | GGC | AAC | CAG | TTC | CAG | AAG | GCT | CAA | GCC | ATC | TCT |
| | | | | 200 | | | | | | | | | | | |

| ALA | PHE | HIS | GLU | 60 MET | ILE | GLN | GLN | THR | PHE | ASN | LEU | PHE | SER | 70 THR | LYS |
|---|---|---|---|---|---|---|---|---|---|---|---|---|---|---|---|
| GCC | TTC | CAT | GAG | ATG | ATC | CAG | CAG | ACC | TTC | AAT | CTC | TTC | AGC | ACA | AAG |
| | | | | | 250 | | | | | | | | | | |

| ASP | SER | SER | ALA | ALA | TRP | ASP | GLU | 80 THR | LEU | LEU | ASP | LYS | PHE | TYR | ILE |
|---|---|---|---|---|---|---|---|---|---|---|---|---|---|---|---|
| GAT | TCA | TCT | GCT | GCT | TGG | GAT | GAG | ACC | CTC | CTA | GAC | AAA | TTC | TAC | ATT |
| | | | | | 300 | | | | | | | | | | |

| GLU | LEU | 90 PHE | GLN | GLN | LEU | ASN | ASP | LEU | GLU | ALA | CYS | 100 VAL | THR | GLN | GLU |
|---|---|---|---|---|---|---|---|---|---|---|---|---|---|---|---|
| GAA | CTT | TTC | CAG | CAA | CTG | AAT | GAC | CTA | GAA | GCC | TGT | GTG | ACA | CAG | GAG |
| | | | | | | 350 | | | | | | | | | |

| VAL | GLY | VAL | GLU | GLU | ILE | 110 ALA | LEU | MET | ASN | GLU | ASP | SER | ILE | LEU | ALA |
|---|---|---|---|---|---|---|---|---|---|---|---|---|---|---|---|
| GTT | GGG | GTG | GAA | GAG | ATT | GCC | CTG | ATG | AAT | GAG | GAC | TCC | ATC | CTG | GCT |
| | | | | | | | 400 | | | | | | | | |

| 120 VAL | ARG | LYS | TYR | PHE | GLN | ARG | ILE | THR | LEU | 130 TYR | LEU | MET | GLY | LYS | LYS |
|---|---|---|---|---|---|---|---|---|---|---|---|---|---|---|---|
| GTG | AGG | AAA | TAC | TTT | CAA | AGA | ATC | ACT | CTT | TAT | CTG | ATG | GGG | AAG | AAA |
| | | | | | | | 450 | | | | | | | | |

| TYR | SER | PRO | CYS | 140 ALA | TRP | GLU | VAL | VAL | ARG | ALA | GLU | ILE | MET | 150 ARG | SER |
|---|---|---|---|---|---|---|---|---|---|---|---|---|---|---|---|
| TAC | AGC | CCT | TGT | GCC | TGG | GAG | GTT | GTC | AGA | GCA | GAA | ATC | ATG | AGA | TCC |

16

FIG. 4 CONTINUED

| | | | | | | | | 160 | | | | | | 166 | |
|---|---|---|---|---|---|---|---|---|---|---|---|---|---|---|---|
| PHE | SER | PHE | SER | THR | ASN | LEU | GLN | LYS | GLY | LEU | ARG | ARG | LYS | ASP | STOP |
| TTC | TCT | TTT | TCA | ACA | AAC | TTG | CAA | AAA | GGA | TTA | AGA | AGG | AAG | GAT | TGA |
| | | | | | | | | | 550 | | | | | | |

AAACT

FIGURE 5

```
                                          1
                                         CYS  ASP  LEU  PRO  GLN  THR
                                         TGT  GAT  CTG  CCT  CAG  ACT


                        10                                         20
  HIS  SER  LEU  GLY  ASN  ARG  ARG  ALA  LEU  ILE  LEU  LEU  ALA  GLN  MET  GLY
  CAC  AGC  CTG  GGT  AAT  AGG  AGG  GCC  TTG  ATA  CTC  CTG  GCA  CAA  ATG  GGA


                              30
  ARG  ILE  SER  HIS  PHE  SER  CYS  LEU  LYS  ASP  ARG  TYR  ASP  PHE  GLY  PHE
  AGA  ATC  TCT  CAT  TTC  TCC  TGC  CTG  AAG  GAC  AGA  TAT  GAT  TTC  GGA  TTC


       40                                             50
  PRO  GLN  GLU  VAL  PHE  ASP  GLY  ASN  GLN  PHE  GLN  LYS  ALA  GLN  ALA  ILE
  CCC  CAG  GAG  GTG  TTT  GAT  GGC  AAC  CAG  TTC  CAG  AAG  GCT  CAA  GCC  ATC


                         60                                              70
  SER  ALA  PHE  HIS  GLU  MET  ILE  GLN  GLN  THR  PHE  ASN  LEU  PHE  SER  THR
  TCT  GCC  TTC  CAT  GAG  ATG  ATC  CAG  CAG  ACC  TTC  AAT  CTC  TTC  AGC  ACA


                                        80
  LYS  ASP  SER  SER  ALA  ALA  TRP  ASP  GLU  THR  LEU  LEU  ASP  LYS  PHE  TYR
  AAG  GAT  TCA  TCT  GCT  GCT  TGG  GAT  GAG  ACC  CTC  CTA  GAC  AAA  TTC  TAC


                   90                                   100
  ILE  GLU  LEU  PHE  GLN  GLN  LEU  ASN  ASP  LEU  GLU  ALA  CYS  VAL  THR  GLN
  ATT  GAA  CTT  TTC  CAG  CAA  CTG  AAT  GAC  CTA  GAA  GCC  TGT  GTG  ACA  CAG


                              110
  GLU  VAL  GLY  VAL  GLU  GLU  ILE  ALA  LEU  MET  ASN  GLU  ASP  SER  ILE  LEU
  GAG  GTT  GGG  GTG  GAA  GAG  ATT  GCC  CTG  ATG  AAT  GAG  GAC  TCC  ATC  CTG


  120                                     130
  ALA  VAL  ARG  LYS  TYR  PHE  GLN  ARG  ILE  THR  LEU  TYR  LEU  MET  GLY  LYS
  GCT  GTG  AGG  AAA  TAC  TTT  CAA  AGA  ATC  ACT  CTT  TAT  CTG  ATG  GGG  AAG


                        140                                        150
  LYS  TYR  SER  PRO  CYS  ALA  TRP  GLU  VAL  VAL  ARG  ALA  GLU  ILE  MET  ARG
  AAA  TAC  AGC  CCT  TGT  GCC  TGG  GAG  GTT  GTC  AGA  GCA  GAA  ATC  ATG  AGA


                                   160                              166
  SER  PHE  SER  PHE  SER  THR  ASN  LEU  GLN  LYS  GLY  LEU  ARG  ARG  LYS  ASP
  TCC  TTC  TCT  TTT  TCA  ACA  AAC  TTG  CAA  AAA  GGA  TTA  AGA  AGG  AAG  GAT


  STOP  AAACT
  TGA
```

EP 0 154 186 B1

FIGURE 6

                    EcoRI
ATG ACC ATG ATT ACG AAT TCC CGG GGA TCC GTC GAC CTG CAG CCA AGC TTG GCA CTG GCC